(19) Europäisches Patentamt
European Patent Office
Office européen des brevets

(11) EP 2 080 807 B1

(12) EUROPÄISCHE PATENTSCHRIFT

(45) Veröffentlichungstag und Bekanntmachung des Hinweises auf die Patenterteilung:
**04.01.2017 Patentblatt 2017/01**

(51) Int Cl.:
***C12P 7/62*** *(2006.01)* ***C12M 1/40*** *(2006.01)*

(21) Anmeldenummer: **08169389.7**

(22) Anmeldetag: **19.11.2008**

(54) **Verfahren zur enzymatischen Herstellung von Carbonsäureestern**

Method for enzymatic manufacture of carboxylic acid esters

Procédé destiné à la préparation enzymatique d'ester d'acid carboxylique

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **16.01.2008 DE 102008004726**

(43) Veröffentlichungstag der Anmeldung:
**22.07.2009 Patentblatt 2009/30**

(73) Patentinhaber: **Evonik Degussa GmbH**
**45128 Essen (DE)**

(72) Erfinder:
- **Thum, Oliver Dr.**
**40880 Ratingen (DE)**
- **Hilterhaus, Lutz Dr.**
**17491 Greifswald (DE)**
- **Liese, Andreas Prof. Dr.**
**21077 Hamburg (DE)**

(74) Vertreter: **Lang, Arne et al**
**Evonik Industries AG**
**Intellectual Property Management**
**Bau 1042A / PB 15**
**Paul-Baumann-Straße 1**
**45764 Marl (DE)**

(56) Entgegenhaltungen:
**EP-A- 0 413 307**
**EP-A- 0 670 372**
**WO-A2-03/070873**
**DE-A1- 2 553 649**
**DE-A1- 10 122 551**
**US-A- 5 713 965**
**US-A1- 2009 181 439**
**US-B2- 7 026 433**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein neuartiges Verfahren zur enzymatischen Herstellung von Carbonsäureestern.

Stand der Technik

Carbonsäureester stellen eine bedeutende Gruppe chemischer Verbindungen dar. Ester von Fettsäuren werden zum Beispiel häufig als Ölphase in kosmetischen Formulierungen eingesetzt. Zur Herstellung solcher Verbindungen ausgehend von Carbonsäuren sind vielfältige Methoden bekannt. Eine erste Übersicht hierzu findet sich bei: Becker u.a., "Organikum", 22. Auflage, Wiley-VCH: 2004, Weinheim, Deutschland, Seite 472 ff.

In letzter Zeit finden immer mehr Verfahren Einsatz, die auf der Verwendung von Biokatalysatoren beruhen. Der Hauptvorteil dieser Verfahren liegt üblicherweise in der Mildheit der Reaktionsbedingungen, die eine schonende Umsetzung bei relativ niedrigen Temperaturen, üblicherweise unter 100°C, erlauben. Dabei kann sich häufig auch ein weiterer Vorteil der enzymatischen Verfahren zeigen, nämlich ihre oft hohe Chemo-, Regio- oder Enantioselektivität. Die Verwendung heterogener Biokatalysatoren, beispielsweise auf inerte Träger immobilisierte Enzyme oder Enzyme enthaltende Mikroorganismen, hat den Vorteil, dass diese nach der Reaktion einfach abgetrennt und gegebenenfalls wiederverwendet werden können. Als Träger für die Enzymimmobilisierung werden häufig Ionenaustauscherharze oder Polymerpartikel eingesetzt, die über geeignete Korngrößenverteilungen verfügen. Beispiele hierzu sind die Marktprodukte Novozym 435, Lipozym RM IM oder Lipozym TL IM der Firma Novozymes A/S, Bagsvaerd, Dänemark oder Amano PS der Firma Amano, Japan. Aber auch viele andere Methoden zur Gewinnung immobilisierter Enzympräparate sind beschrieben, beispielsweise in K. Faber, "Biotransformations in Organic Chemistry", Springer: 2000, Berlin, Deutschland, 384ff., J. Am. Chem. Soc. 1999, 121, 9487-9496, J. Mol. Catal. B, 2005, 35, 93-99 oder in der Patentanmeldung DE 10 2007 031689.7 oder in EP 0413307.

Eine weitere Bedingung zur Gewährleistung der Wiederverwendbarkeit ist die Minimierung der mechanischen Kräfte, die auf die eingesetzten Katalysatoren wirken und eine Desintegration des Trägers und somit eine deutliche Teilchengrößenreduzierung verursachen, wodurch die Anforderung der leichten Abtrennbarkeit nicht mehr erfüllt ist und oftmals auch große Aktivitätsverluste zu beobachten sind. Solche Kräfte treten vor allem auf, wenn konventionelle Rührreaktoren verwendet werden. Beispielsweise beschreibt DE 10 2007 031689.7 die auftretende Korngrößenreduktion von Enzymimmobilisaten nach Verwendung im Rührkolben.

Eine Möglichkeit, die mechanische Stabilität der eingesetzten Katalysatoren zu verbessern liegt in dem Einsatz eines Festbettreaktors. Eur. J. Lipid Sci. Technol. 2003, 105, 601-607 beschreibt zum Beispiel den Einsatz eines Festbettreaktors zur Durchführung Lipase-katalysierter Veresterungen. Hier wird der Katalysator in Form eines gepackten Betts eingesetzt und die Reaktionsmischung aus einem Vorratsbehälter im Kreislauf durch das Festbett gepumpt, bis der gewünschte Umsatzgrad erreicht ist.

Die zum Erhalt möglichst hoher Produktqualität erwünschte Reduktion der Reaktionstemperatur hat allerdings auch Nachteile für die technische Umsetzung solcher Reaktionen. Bei der Herstellung von Carbonsäureestern aus den entsprechenden Säuren und Alkoholen handelt es sich um Gleichgewichtsreaktionen, bei denen ein Produkt, üblicherweise das entstehende Reaktionswasser, aus dem System abgetrennt werden muss, um hohe Umsätze zu erreichen. Zum Erreichen besonders hoher Umsatzgrade, z. B. von über 99%, ist eine nahezu vollständige Entfernung des Wassers notwendig. Dies erfolgt üblicherweise destillativ.

Es ist dem Fachmann geläufig, dass die destillative Entfernung von Substanzen aus Mischungen mit abnehmender Konzentration der zu entfernenden Substanzen zunehmend schwieriger wird, da der Dampfdruck der Substanz in Mischungen gegenüber dem in Reinform deutlich erniedrigt wird ("Raoult' sches Gesetz"). Daher sind zur effektiven Wasserentfernung besondere Methoden nötig.

Eine thermodynamisch kontrollierte Möglichkeit besteht in der Erhöhung der Temperatur der Mischung, beispielsweise auf deutlich über 100°C. Diese Methode entfällt allerdings bei enzymatischen Verfahren, da zum einen der eigentliche Vorteil dieses Verfahrens, nämlich die Reaktionsführung bei niedrigen Temperaturen, zunichte gemacht wird, und zum anderen, weil die verwendeten Enzyme üblicherweise bei solch hohen Temperaturen eine signifikant verkürzte Haltbarkeit aufweisen.

Eine weitere thermodynamisch kontrollierte Möglichkeit besteht darin, den Umgebungsdruck möglichst nahe an den Dampfdruck des zu entfernenden Wassers in der Mischung anzunähern. Das dafür benötigte Hochvakuum ist allerdings im Produktionsmaßstab, also in Anlagen mit mehreren Tonnen Fassungsvermögen, nur mit sehr großem Aufwand realisierbar.

Eine weitere Alternative stellt die Verwendung von Lösungsmitteln dar, mit denen zum einen der Dampfdruck des Wassers erhöht werden kann oder zum anderen das Wasser azeotrop abdestilliert werden kann. Die Verwendung von Lösungsmitteln aber ist üblicherweise unerwünscht, da dies zum einen zusätzliche Kosten verursacht (niedrigere Reaktorbeladung, Recyclingkosten, etc.) und zum anderen für viele Produktanwendung, z. B. in der kosmetischen oder pharmazeutischen Industrie, die Abwesenheit von Lösungsmittelresten gewährleistet werden muss, oftmals bis in den

einstelligen ppm Bereich. Letzteres ist zwar meist prinzipiell möglich, erfordert aber üblicherweise zusätzliche Aufarbeitungsschritte, wie z. B. Destillation, Extraktion oder Dämpfen, die zusätzliche Zeit in Anspruch nehmen, und daher die Herstellkosten weiter erhöhen und die Raum-Zeit-Ausbeuten der Verfahren senken.

Eine weitere Variante besteht darin, die kinetischen Limitierungen des Stoffüberganges von der Reaktionsmischung in den Gasraum zu minimieren, indem zum Beispiel die Oberfläche der Mischung vergrößert wird, beispielsweise bei dem Einsatz von Fallfilmverdampfern. Auch hier ist der apparative Aufwand oft unverhältnismäßig hoch.

J. Biotech. 2004, 110, 209-217 beschreibt die enzymatische Esterspaltung zur Synthese von Ketoprofen in einem begasten Reaktor. Da die Reaktion in Wasser stattfindet wird allerdings nicht Wasser abgetrennt, das üblicherweise besonders schwer aus organischen Medien zu entfernen ist, sondern 2-Chlorethanol. Allerdings wurden nur Umsätze von unter 40% erreicht. Bei diesen niedrigen Umsätzen ist durch den großen Überschuss von Wasser das Gleichgewicht noch nicht erreicht, so dass aus thermodynamischen Gründen überhaupt keine Produktentfernung notwendig ist, daher also auch keinen Einfluß auf die Reaktionsführung hat. Außerdem stellen die Autoren einen deutlichen Aktivitätsverlust nach der dritten Verwendung des Enzyms dar (siehe Abbildung 6 auf Seite 215, a. a. O.), so dass der Anspruch der guten Wiederverwertbarkeit nicht erfüllt ist. Die initial beobachtete höhere Reaktionsgeschwindigkeit des in dem begasten Reaktor im Vergleich zum Festbettreaktor erklärt sich durch die um ein Drittel erhöhte Enzymkonzentration (5 mmol Ester bei 50 mg Enzym im Vergleich zu 40 mmol Ester bei 300 mg Enzym, siehe Fußnote zur Abbildung 6 auf Seite 215, a. a. O.); die erhöhte Produktivität durch Abbruch der begasten Reaktionen bei deutlich niedrigeren Umsatzraten und der damit einhergehenden Ausnutzung der höheren Anfangsgeschwindigkeiten.

Bei der technischen Durchführung von Veresterungsreaktionen ist es meist wünschenswert, die Reaktionszeit zu minimieren, um die Herstellkosten zu minimieren. Üblicherweise sollen Gesamtreaktionszeiten unter 24, bevorzugt unter 12, besonders bevorzugt unter 6, insbesondere unter 3 Stunden liegen. Dazu ist es notwenig, ein Verständnis über die geschwindigkeitsbestimmenden Faktoren der zu untersuchenden Reaktion zu erhalten. Im Falle der Veresterungsreaktion kann dies die Menge an eingesetztem Katalysator oder, wie oben dargelegt, die Entfernung des Reaktionswassers sein. In ersterem Falle schafft eine Erhöhung der Katalysatormenge Abhilfe, im zweiten Fall eine Optimierung der Wasseraustragung.

Nach dem oben beschriebenen Stand der Technik ergeben sich üblicherweise technische Verfahren zur enzymatischen Veresterung, bei denen Enzymmengen eingesetzt werden, die eine Reaktionszeit von unter 24, bevorzugt unter 12, besonders bevorzugt unter 6, insbesondere unter 3 Stunden theoretisch erlauben sollten, aufgrund der Limitierung der Wasseraustragung aber tatsächlich deutlich länger dauern.

Als Maß für die Effizienz der Reaktion kann der Vergleich der tatsächlich beobachteten, effektiven Reaktionszeit (ER) zur Erreichung eines bestimmten Umsatzgrades mit einer berechneten, theoretischen Mindestreaktionszeit (TR) dienen. Letztere kann aus der gemessenen spezifischen Aktivität des eingesetzten Enzyms in Verbindung mit der Einsatzmenge und der Michaelis-Menten Konstante $K_M$ dieses Enzyms für die betreffende Reaktion in erster Näherung berechnet werden. Die theoretischen Hintergründe finden sich beispielsweise in: Voet, Voet, "Biochemie", 1. Auflage, Wiley-VCH: 1992, Weinheim, Deutschland, p. 329-331. Zur Veranschaulichung der Effizienz des Verfahrens kann der Quotient aus der effektiven Reaktionszeit ER und der theoretischen Mindestreaktionszeit TR gebildet werden. Dieses Effektivitätsverhältniss (EV) ist ein gut geeignetes und im Folgenden verwendetes Maß für die Effizienz enzymatischer Veresterungsverfahren.

Die theoretische Herleitung der Mindestreaktionszeit TR und des Effektivitätsverhältnisses EV ist im Abschnitt "Materialien und Methoden" ausführlich beschrieben.

Im Idealfall ist EV gleich 1, für durchzuführende reale Reaktionen ist es erstrebenswert, EV möglichst nahe an 1 zu senken. Besonders bei hohen Umatzraten, beispielsweise größer 99%, sind jedoch die beobachteten Effektivitätsverhältnisse deutlich größer als 1, oftmals sogar größer als 5 oder größer als 10. Die nicht erfindungsgemäßen Beispiele 1 und 2 zeigen in Verbindung mit der entsprechenden Berechnung der EVs in Beispiel 6 die sehr hohen EVs der Verfahren des Standes der Technik.

Es besteht daher weiterhin Bedarf an Reaktionsverfahren zur enzymatischen Herstellung von Carbonsäureestern, welche mindestens einen der Nachteile des Standes der Technik überwinden.

<u>Aufgabe der Erfindung</u>

Aufgabe der vorliegenden Erfindung war daher die Bereitstellung eines universellen Verfahrens zur lösungsmittelfreien, enzymatischen Herstellung von Carbonsäureestern, ausgehend von Carbonsäuren und Alkoholen, welches sehr hohe Umsätze bei kurzen Reaktionszeiten und gleichzeitig eine hohe Zahl von Wiederverwendungen ermöglicht.

Weitere nicht explizit genannte Aufgaben ergeben sich aus dem Kontext der nachfolgenden Beschreibung, den Beispielen sowie den Ansprüchen.

Überraschender Weise wurde gefunden, dass diese Aufgabe durch ein Reaktorkonzept erfüllt wird, bei dem sowohl die Durchmischung der Reaktanden als auch die Austragung des Reaktionswassers durch Einleiten eines Gases erreicht wird.

Gegenstand der vorliegenden Erfindung ist daher ein Verfahren zur Herstellung von Carbonsäurederivaten, welches dieses Reaktorkonzept nutzt.

Beschreibung der Erfindung

In dem erfindungsgemäßen Verfahren wird die eingesetzte Reaktionsmischung nebst Biokatalysator durch Einleiten eines Gasstroms durchmischt und gleichzeitig von entstandenem Reaktionswasser befreit.

Da durch das erfindungsgemäße Verfahren im Reaktionsraum auf den Einsatz von Rührwerken verzichtet werden kann, und da die Durchmischung durch die Gaszufuhr gewährleistet ist, ist der mechanische Stress, aber auch die durch den Rührer induzierte thermische Belastung auf die Katalysatorteilchen auf ein Minimum reduziert. Dies sind Grundvoraussetzungen dafür, dass der Katalysator wiederverwendet werden kann.
Erfindungsgemäß können als Gase solche verwendet werden, die mit den Reaktanden, dem Katalysator oder den Reaktormaterialien keine Reaktionen eingehen. Bevorzugt eingesetzt werden Luft, Magerluft, Sauerstoff, Stickstoff, Edelgase oder Kohlendioxid. Die eingesetzten Gase können aus geeigneten Druckbehältern, z. B. Gasflaschen, oder durch Kompressoren zugeführt werden. Der Abluftstrom kann abgeleitet oder durch geeignete Maßnahmen wiederverwendet und im Kreislauf geführt werden.

Als Katalysatoren können erfindungsgemäß solche verwendet werden, deren mittlere Korngröße so beschaffen ist, dass sie mit den üblichen verfügbaren Filtersystemen ohne großen Druckverlust in dem Reaktionsbehälter zurückgehalten werden können, also größer 0,5 μm, bevorzugt größer 5 μm, besonders bevorzugt größer 10 μm, insbesondere größer 25 μm.

Zur Herstellung der Enzymimmobilisate können ganze Zellen, ruhende Zellen, gereinigte Enzyme oder Zellextrakte, die die entsprechenden Enzyme enthalten oder Mischungen davon eingesetzt werden. Bevorzugt eingesetzt werden hydrolytische Enzyme, z. B. Lipasen, Esterasen oder Proteasen, wie beispielsweise Lipasen aus *Candida rugosa, Candida antarctica, Pseudomonas sp., Thermomyces langosiosus,* Schweinepankreas, *Mucor miehei, Alcaligines sp.,* Cholesterolesterase aus *Candida rugosa,* Esterase aus der Schweineleber, besonders bevorzugt Lipasen, eingesetzt. Demgemäß weisen die Enzymimmobilisate vorzugsweise Enzyme aus der Klasse der Hydrolasen, bevorzugt Lipasen, auf.

Als Träger zur Immobilisierung der Enzyme oder Enzyme enthaltenden Mikroorganismen können inerte organische oder anorganische Träger verwendet werden. Vorzugsweise werden als inerte Träger solche partikulären Träger verwendet bzw. sind im Enzymimmobilisat vorhanden, die eine Teilchengrößenverteilung aufweisen, bei der mindestens 90% der Teilchen eine Teilchengröße von 0,5 bis 5000 μm, bevorzugt von 10 μm bis 2000 μm, besonders bevorzugt von 25 μm bis 2000 μm aufweisen. Als organische Träger können insbesondere solche eingesetzt werden, die Polyacrylat, Polymethacrylat, Polyvinylstyrol, Styrol-Divinylbenzolcopolymeren, Polypropylen, Polyethylen, Polyethylenperepthalat, PTFE und/oder andere Polymere aufweisen oder aus diesen bestehen. Als Trägermaterial können, in Abhängigkeit von dem zu immobilisierenden Enzym, insbesondere saure oder basische Ionenaustauscherharze eingesetzt werden, beispielsweise Duolite A568, Duolite XAD 761, Duolite XAD 1180, Duolite XAD 7HP, Amberlite IR 120, Amberlite IR 400, Amberlite CG 50, Amberlyst 15 (alles Produkte der Fa. Rohm und Haas) oder Lewatit CNP 105 und Lewatit VP OC 1600 (Produkte der Fa. Lanxess, Leverkusen, Deutschland). Als anorganische Träger können aus dem Stand der Technik bekannte, oxidische und/oder keramische Träger eingesetzt werden. Insbesondere können als anorganische Träger beispielsweise Celite, Zeoliten, Silica, Controlled-Pore Glas (CPG) oder andere Träger, wie sie zum Beispiel in L. Cao, "Carrier-bound Immobilized Enzymes: Principles, Application and Design", Wiley-VCH: 2005, Weinheim, Deutschland, beschrieben sind, eingesetzt werden. Besonders bevorzugt bestehen die im Enzymimmobilisat vorhandenen inerten Träger bzw. die zur Herstellung der Enzymimmobilisate verwendeten inerten Träger aus Polyvinylstyrol, Polymethacrylat oder Polyacrylat.

Gegenstand der vorliegenden Erfindung sind somit Verfahren zur enzymatischen Synthese von Carbonsäureestern, wobei das Durchmischen und der Austrag des Reaktionswassers durch Einleiten eines Gases erfolgen, unter Erreichung niedriger Effektivitätsverhältnisse EV.

Ein weiterer Gegenstand ist ein Verfahren bei dem der enzymatische Katalysator partikulär auf einem Trägermaterial kovalent oder nicht-kovalent fixiert ist.

Ein weiterer Gegenstand ist ein Verfahren bei dem das Trägermaterial eine mittlere Korngröße von größer als 0,5 μm aufweist.

Ein weiterer Gegenstand ist ein Verfahren bei dem als Gas Luft, Magerluft, Sauerstoff, Stickstoff, Edelgase oder Kohlendioxid, bevorzugt Luft oder Stickstoff, eingesetzt werden.

Ein weiterer Gegenstand ist ein Verfahren bei dem die Reaktion bei einer Temperatur von 20 °C bis 100 °C durchgeführt wird.

Ein weiterer Gegenstand ist ein Verfahren bei dem eine Umsatzrate von 95% mit einem Effektivitätsverhältnis $EV_{95}$ < 3,0 erreicht wird.

Ein weiterer Gegenstand ist ein Verfahren bei dem 98% Umsatz mit einem Effektivitätsverhältnis $EV_{98}$ < 4,0

erreicht wird.

Ein weiterer Gegenstand ist ein Verfahren bei dem 99% Umsatz mit einem Effektivitätsverhältnis $EV_{99}$ < 5,0 erreicht wird.

Ein weiterer Gegenstand ist ein Verfahren bei dem 99,6% Umsatz mit einem Effektivitätsverhältnis $EV_{99,6}$ < 8,0 erreicht wird.

Die Immobilisierung des enzymatischen Katalysators auf den Trägerpartikeln kann erfindungsgemäß kovalent oder nichtkovalent erfolgen.

Beispiele für die erfindungsgemäß eingesetzten Enzymimmobilisate sind Novozym 435, Lipozym RM IM oder Lipozym TL IM der Firma Novozymes A/S, Bagsvaerd, Dänemark oder Amano PS der Firma Amano, Japan.

Das erfindungsgemäße Verfahren wird bei einer Temperatur von 20 °C bis 100 °C, bevorzugt von 40 °C bis 90 °C durchgeführt.

Es zeigt sich, dass durch das erfindungsgemäße Verfahren bei Veresterungsreaktionen ein Umsatz von 95% mit einem Effektivitätsverhältnis $EV_{95}$ < 3,0, bevorzugt < 2,5, besonders bevorzugt < 2,0, insbesondere < 1,75 erreicht werden kann.

Weiterhin zeigt sich, dass durch das erfindungsgemäße Verfahren bei Veresterungsreaktionen ein Umsatz von 98% mit einem Effektivitätsverhältnis $EV_{98}$ < 4,0, bevorzugt < 3,00, besonders bevorzugt < 2,50 erreicht werden kann.

Außerdem zeigt sich, dass durch das erfindungsgemäße Verfahren bei Veresterungsreaktionen ein Umsatz von 99% mit einem Effektivitätsverhältnis $EV_{99}$ < 5,0, bevorzugt < 4,0, besonders bevorzugt < 3,0, insbesondere < 2,5 erreicht werden kann.

Schließlich zeigt sich, dass durch das erfindungsgemäße Verfahren bei Veresterungsreaktionen ein Umsatz von 99,6% mit einem Effektivitätsverhältnis $EV_{99,6}$ < 8,0, bevorzugt < 5,0, besonders bevorzugt < 4,0, insbesondere < 3,5 erreicht werden kann.

Erfindungsgemäß wird das Verfahren bevorzugt eingesetzt zur Durchführung von Umsetzungen der allgemeinen Formel I

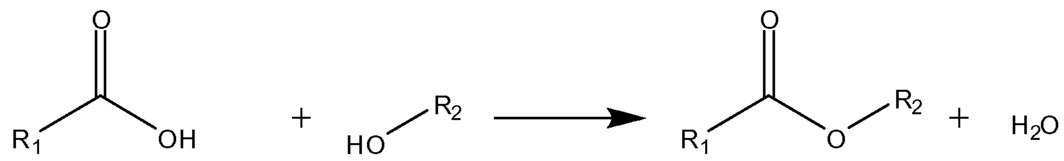

I

wobei

> $R_1$ der Acylrest linearer, verzweigter, gesättigter oder ungesättigter, gegebenenfalls zusätzlich substituierter Carbonsäuren mit 2 bis 30 C-Atomen,
>
> $R_2$ ein linearer, verzweigter, gesättigter oder ungesättigter, gegebenenfalls zusätzlich substituierter Alkylrest mit 2 bis 30 C-Atomen ist.

Erfindungsgemäß können die optionalen zusätzlichen Substituenten der Reste $R_1$ und $R_2$ unabhängig voneinander beispielsweise Hydroxygruppen, Estergruppen, Polyestergruppen, Carbonatgruppen, Polycarbonatgruppen, Ethergruppen, Polyethergruppen, Urethangruppen, Polyurethangruppen, Amidgruppen, Polyamidgruppen, Alkylamingruppen, Dialkylamingruppen, Halogenide, Siloxangruppen, Estergruppen anorganischer Säuren, Sulfate, Phosphate oder ähnliche Gruppierungen sein.

In einer bevorzugten Ausführungsform stellt $R_1$ den Acylrest handelsüblichen Säuren, wie beispielsweise Essigsäure, Propansäure, Butansäure, Pentansäure, Chloressigsäure, Trifluoressigsäure, Ethylhexansäure, Isononansäure, Isotridecansäure oder Isostearinsäure dar.

In einer weiteren bevorzugten Ausführungsform werden als $R_1$ Acylreste natürliche Fettsäuren auf Basis natürlicher pflanzlicher oder tierischer Öle eingesetzt. Bevorzugt werden natürliche Fettsäuren wie z.B. Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, Linolensäure, Eicosapentaensäure, Docosahexaensäure oder Arachidonsäure, allein oder in Mischung eingesetzt. Der Rest $R_1$ kann ebenfalls der Acylrest von Polykondensationsprodukten von

hydroxyfunktionalisierten Säuren, beispielsweise Poly-12-hydroxystearinsäure oder Polyricinolsäure sein. Bei dem als Rest $R_1$ eingesetzten Acylresten kann es sich um technische Mischungen handeln, beispielsweise um Mischungen von natürlichen Fettsäuren, z. B. Rapsölfettsäure, Sojaölfettsäure, Sonnenblumenölfettsäure, Talgölfettsäure, Palmölfettsäure, Palmkernölfettsäure, Kokosfettsäure, die in Abhängigkeit von ihrer konkreten Quelle und den eingesetzten Aufreinigungsverfahren in ihrer genauen Zusammensetzung Schwankungen unterworfen sein können, sowie typische Nebenbestandteile, wie ungesättigte, funktionalisierte oder verzweigte Komponenten enthalten können. Darüber hinaus können auch Mischungen von Säuren anderer Herkunft, zum Beispiel auf Basis petrochemischer Verfahren, eingesetzt werden.

In einer weiteren bevorzugten Ausführungsform werden als $R_2$ beispielsweise die Kohlenwasserstoffreste von Propanol, Butanol, Pentanol, Hexanol, Octanol oder deren Isomeren wie Isopropanol, Isobutanol, 2-Ethylhexanol, Isononylalkohol, Isotridecylalkohol, mehrwertige Alkohole, wie 1,6-Hexandiol, 1,2-Pentandiol, Dihydroxyaceton, 1,2-Propylenglykol, 1,3-Propylenglykol, Neopentylglykol, Trimethylolpropan, Pentaerithrol, Sorbitol, Glycerin, Diglycerin, Triglycerin, Polyglycerin, Ethylenglykol, Diethylenglykol, Triethylenglykol, Polyethylenglykol, oder aminofunktionalisierte Alkohole, wie N,N-Dimethylethanolamin, eingesetzt. Weitere Beispiele sind die Kohlenwasserstoffreste von Alkoholen, die nach bekannten Verfahren aus einbasischen Fettsäuren auf Basis natürlicher pflanzlicher oder tierischer Öle mit 6 bis 30 Kohlenstoffatomen, insbesondere mit 8 bis 22 Kohlenstoffatomen, hergestellt werden, beispielsweise Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, Linolensäure, Eicosapentaensäure, Docosahexaensäure oder Arachidonsäure, allein oder in Mischung eingesetzt.

Beispiele für die erfindungsgemäßen polyethersubstituierten Reste $R_2$ sind die Alkylreste von Polyethylenglykol, Polypropylenglykol, Polybutylenglykol, Polystryroloxid oder Copolymere von mindestens zwei Monomeren aus der Gruppe Ethylenglykol, Propylenglykol, Butylenglykol Stryroloxid, sowie Polyglycerin. Diese polyethersubstituierten Reste können ihrerseits wieder Substituenten an weiteren OH-Gruppen tragen, beispielsweise Alkyl- oder Alkenylether oder Alkylester.

Beispiele für die erfindungsgemäßen polyestersubstituierten Reste $R_2$ sind freie OH-Gruppen tragende Polymere auf Basis von $\varepsilon$-caprolacton oder $\gamma$-Valerolacton, beispielsweise Placcel L212AL der Firma Daicel.

Beispiele für die erfindungsgemäßen polycarbonatsubstituierten Reste $R_3$ sind freie OH-Gruppen tragende Polymere auf Basis von Dialkylcarbonat- und Dihydroxyalkyleinheiten, beispielsweise Placcel CD220 der Firma Daicel.

Beispiele für die erfindungsgemäßen polysiloxansubstituierten Reste $R_2$ sind freie Alkyl-OH-Gruppen tragende organomodifizierte Polysiloxane, wie sie beispielsweise durch Hydrosilylierung von SiH-Siloxanen mit terminal ungesättigten Alkoholen nach dem Fachmann bekannten Methoden zugänglich sind. Insbesondere können hier lineare Alkenole, wie 5-Hexen-1-ol oder Allyloxyderivate, wie Allyloxyethanol, Allylglycerin, Allylpolyglycerin, Allyltrimethylolpropan, Allylpolyethylenglykol, Allylpolypropylenglykol, oder Allylpolyethylenglykol-polyproypylenglykol-copolymere, verwendet werden.

Beispiele für die erfindungsgemäßen polysiloxansubstituierten Reste $R_1$ sind freie Carboxygruppen tragende organomodifizierte Polysiloxane, wie sie beispielsweise durch Hydrosilylierung von SiH-Siloxanen mit terminal ungesättigten Säuren nach dem Fachmann bekannten Methoden zugänglich sind. Insbesondere können hier Acrylsäure, Methacrylsäure oder Undec-10-ensäure verwendet werden.

Die nachfolgenden Beispiele und theoretischen Überlegungen sollen die vorliegende Erfindung näher erläutern, ohne den Schutzbereich einzuschränken, der sich aus der Beschreibung und den Patentansprüchen ergibt.

Das erfindungsgemäße Verfahren wird nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Erfindung Verbindungen, wie z. B. polymere Trägermaterialien, Säuren oder Ester, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen, zu verstehen.

Sollten bei den durchgeführten Veresterungsreaktionen die Reaktanden in einem anderen als dem stöchiometrischen Verhältnis verwendet werden, so beziehen sich die angegebenen Umsatzdaten auf die im Unterschuss eingesetzte Komponente.

<u>Materialien und Methoden:</u>

Novozym 435 (NZ435) ist ein kommerzielles Enzymimmobilisat der Firma Novozymes A/S, Bagsvaerd/Dänemark, einer auf einem Polymethyacrylat durch Adsorption immobilisierten Lipase B aus *C. antarctica*.

Bestimmung von $k_{cat}$ für die durch Novozym 435 katalysierte Herstellung von Myristylmyristat

Es wurden 10 mg Novozym 435 in 5 mL äquimolare Substratlösung (Myristinsäure und Myristylalkohol) gegeben und bei 60 °C rührend inkubiert. Proben ($V_{probe}$: 50 μL) wurden über 25 min alle 5 min entnommen und in 950 μL Decan (Interner Standard: 4 mM Dodecan) überführt. Die Bestimmung von $k_{cat}$ erfolgte anhand der initialen Produktbildungsraten und wurde mit 7000 μmol*mg$^{-1}$*min$^{-1}$ bestimmt. Der Nachweis von Myristylmyristat erfolgte gaschromatographisch (Shimadzu 2010, Säule BTX Fa. SGE; Länge 25 m, I.D. 0,22 μm; Film: 0,25 μM; Detektortyp: FID bei 300 °C; Injektortemperatur 275 °C und Injektionsvolumen 1 μL, Splitratio 35.0; Trägergasdruck (Helium) 150 kpa; Temperaturprogramm: Anfangstemperatur 60 °C hold 1,5 min, Temperaturanstieg 20 °C/min, Endtemperatur 250 °C hold 2,5 min).

Bestimmung von $K_M$ für die durch Novozym 435 katalysierte Herstellung von Myristylmyristat

Es wurden 10 mg Novozym 435 in 5 mL verschieden konzentrierte, äquimolare Substratlösung (Myristinsäure und Myristylalkohol, jeweils 10 mM bis 1000 mM in Methylcyclohecan) gegeben und bei 60 °C rührend inkubiert. Proben ($V_{probe}$: 50 μL) wurden über 25 min für jede Konzentration alle 5 min entnommen und in 950 μL Decan (Interner Standard: 4 mM Dodecan) überführt. Die Auftragung der erhaltenen Anfangsgeschwindigkeiten gegen die Substratkonzentration lieferte abgeschätzt eine halbmaximale Reaktionsgeschwindigkeit bei $K_M$ von 150 mM. Der Einfachheit halber wurde die Konstante nicht für jede einzelne Komponente bestimmt, sondern aufgrund des äquimolaren Verbrauchs im Reaktionsverlauf für diese konkrete Mischung bestimmt.

Herleitung des Effektivitätsverhältnisses EV:

Als Modellreaktion wurde die Herstellung von Myristylmyristat unter Verwendung von immobilisierter Lipase B aus C. *antarctica* untersucht. Es ist dem Fachmann geläufig, dass mit sinkender Substratkonzentration die tatsächliche Reaktionsgeschwindigkeit abnimmt. Der mathematische Zusammenhang wird mit der Michaelis-Menten Gleichung beschrieben siehe auch Voet, Voet, "Biochemie", 1. Auflage, Wiley-VCH: 1992, Weinheim, Deutschland, p. 329-331.

Daher ergibt sich Gleichung 1, mit [S] = Substratkonzentration im Gleichgewicht:

$$-\frac{d[S]}{dt} = v = v_{\max} \cdot \frac{[S]}{[S]+K_M} \qquad \text{Gl. 1,}$$

wobei

$$v_{\max} = k_{cat} \cdot c_E \qquad \text{Gl. 2,}$$

mit $C_E$ = Enzymkonzentration. Daraus folgt Gleichung 3:

$$-\frac{d[S]}{dt} = v = k_{cat} \cdot c_E \cdot \frac{[S]}{[S]+K_M} \qquad \text{Gl. 3}$$

Zur Bestimmung des zeitabhängigen Umsatzes lässt sich Gleichung 3 integrieren und liefert Gl.4:

$$TR_n = \frac{[S_0]-[S]}{v_{\max}} + \frac{K_M}{v_{\max}} \cdot \ln\frac{[S_0]}{[S]} \qquad \text{Gl. 4}$$

mit $TR_n$ = Theoretische Mindestreaktionszeit zum Erreichen des Umsatzgrads n, $[S_0]$ = Substratkonzentration bei t = 0 und [S] = Substratkonzentration bei Umsatzgrad n.

Für die Modellreaktion ergibt sich nun beispielsweise bei Einsatz von 0.4 Gewichtsprozent Enzym mit $k_{cat}$ = 7000 μmol*mg$^{-1}$*min$^{-1}$ und $K_M$ = 150 mmol*L$^{-1}$ für einen angestrebten Umsatz von 99, 6% eine $TR_{99,6}$ von 112 Minuten.

Der Vergleich der effektiven Reaktionszeit $ER_n$ der untersuchten Beispiele mit der der Theoretischen Reaktionszeit $TR_n$ liefert nun das Effektivitätsverhältnis $EV_n$ für den Umsatzgrad $U_n$ nach Gleichung 5:

$$EV_n = ER_n/TR_n \qquad Gl.\ 5$$

Beispiele

Beispiel 1 (Nicht erfindungsgemäß) Synthese von

Myristylmyristat im Rührkolben:

329 g Myristylalkohol und 351 g Myristinsäure wurden in einem 1L-Vorlagebehälter auf 60 °C temperiert und durch einen Magnetrührer durchmischt. Es wurden 2,72 g Novozym 435 zugegeben und zur Entfernung des entstehenden Reaktionswassers ein Vakuum von 5 mbar angelegt und das Wasser abdestilliert. Der Reaktionsverlauf wurde für ~24 h über Säurezahltitration verfolgt (vgl. Tabelle 1).

Tabelle 1: Umsatzdaten zu Beispiel 1:

| Zeit [min] | Umsatz [%] |
|---|---|
| 0 | 0,0% |
| 60 | 43,9% |
| 122 | 71,1% |
| 180 | 93,0% |
| 355 | 95,1% |
| 640 | 98,0% |
| 802 | 99,0% |
| 1079 | 99,4% |
| 1440 | 99,6% |

Beispiel 2 (Nicht erfindungsgemäß) Synthese von Myristylmyristat im Festbettreaktor - aufwändig modifiziertes Fallfilmverfahren zur Entfernung des Reaktionswassers:

502 g Myristylalkohol und 513 g Myristinsäure wurden in einem 2L-5-Halskolben mit Bodenauslass auf 60 °C temperiert und mit einem mechanischen Rührer durchmischt. 4,06 g Novozym 435 wurden in ein auf 60°C beheiztes Festbett (2 cm Höhe, 3 cm Durchmesser) gefüllt. Anschließend wurde die Reaktionsmischung mit einer Zahnradpumpe über auf 60°C beheizte Edelstahlrohre im Kreislauf durch das mit Enzym beladenen Festbett zurück in den Kolben gepumpt, die Flußrate wurde auf 50 $mL*min^{-1}$ eingestellt. Zur Entfernung des Reaktionswassers wurde ein Vakuum von 5 mbar angelegt und das Wasser abdestilliert. Zur Beschleunigung der Wasserentfernung wurde die Reaktionsmischung beim Wiedereintritt in den Kolben über eine Strecke von etwa 15 cm in einem dünnen Film an der Innenwand einer auf 60°C temperierten Glassäule herabfließen gelassen. Der Reaktionsverlauf wurde für ~14 h über Säurezahltitration verfolgt (vgl. Tabelle 2).

Tabelle 2: Umsatzdaten zu Beispiel 2:

| Zeit [min] | Umsatz [%] |
|---|---|
| 0 | 0,0% |
| 180 | 81,1% |
| 240 | 91,0% |
| 300 | 96,2% |
| 420 | 98,2% |

(fortgesetzt)

| Zeit [min] | Umsatz [%] |
|---|---|
| 540 | 99,1% |
| 840 | 99,6% |

Beispiel 3 (erfindungsgemäß) Synthese von Myristylmyristat mit Wasseraustrag durch Begasung:

11010 g Myristylalkohol und 11500 g Myristinsäure wurden in einem auf 60° beheizten Glasgefäß mit einem Innendurchmesser von 300 mm und einer Höhe von 700 mm auf 60 °C erhitzt. Durch eine Am Gefäßboden angebrachte Ringbrause wurden 5 $m^3$/h Stickstoff in die Mischung eingetragen. Es wurden 90 g Novozym 435 zugegeben und der Reaktionsverlauf für ~6 h über Säurezahltitration verfolgt (vgl. Tabelle 3).

Tabelle 3: Umsatzdaten zu Beispiel 3:

| Zeit [min] | Umsatz [%] |
|---|---|
| 0 | 0,0% |
| 60 | 67,0% |
| 120 | 92,0% |
| 180 | 96,9% |
| 240 | 98,8% |
| 300 | 99,5% |
| 360 | 99,7% |

Beispiel 4 (erfindungsgemäß) Synthese von Myristylmyristat mit Wasseraustrag durch Begasung:

55305 g Myristylalkohol und 56705 g Myristinsäure wurden in einem auf 60° beheizten Glasgefäß mit einem Innendurchmesser von 300 mm und einer Höhe von 2500 mm auf 60 °C erhitzt. Durch eine am Gefäßboden angebrachte Ringbrause wurden 5 $m^3$/h Luft in die Mischung eingetragen. Es wurden 448 g Novozym 435 zugegeben und der Reaktionsverlauf für ~9 h über Säurezahltitration verfolgt (vgl. Tabelle 4).

Tabelle 4: Umsatzdaten zu Beispiel 4:

| Zeit [min] | Umsatz [%] |
|---|---|
| 0 | 0,0% |
| 30 | 35,4% |
| 60 | 49,9% |
| 90 | 65,0% |
| 120 | 74,5% |
| 165 | 83,9% |
| 180 | 85,4% |
| 210 | 89,1% |
| 240 | 91,3% |
| 300 | 96,6% |
| 360 | 98,3% |
| 420 | 99,2% |

(fortgesetzt)

| Zeit [min] | Umsatz [%] |
|---|---|
| 480 | 99,5% |
| 540 | 99,8% |

Beispiel 5 (erfindungsgemäß) Synthese von Myristylmyristat mit Wasseraustrag durch Begasung:

55305 g Myristylalkohol und 56780 g Myristinsäure wurden in einem auf 60° beheizten Glasgefäß mit einem Innendurchmesser von 300 mm und einer Höhe von 2500 mm auf 60 °C erhitzt. Durch eine am Gefäßboden angebrachte Ringbrause wurden 13,5 $m^3/h$ Luft in die Mischung eingetragen. Es wurden 448 g Novozym 435 zugegeben und der Reaktionsverlauf für ~7 h über Säurezahltitration verfolgt (vgl. Tabelle 5).

Tabelle 5: Umsatzdaten zu Beispiel 5:

| Zeit [min] | Umsatz [%] |
|---|---|
| 0 | 0,0% |
| 30 | 34,0% |
| 65 | 62,2% |
| 95 | 76,2% |
| 125 | 85,7% |
| 155 | 92,7% |
| 185 | 95,8% |
| 225 | 98,0% |
| 275 | 98,8% |
| 335 | 99,4% |
| 395 | 99,7% |

Beispiel 6 (erfindungsgemäß) Bestimmung der $EV_n$ für die Beispiele 1-5:

Für Beispiele 1-5 wurde die Zeit bestimmt (ggf. durch Interpolation), die zur Erreichung von 95%, 98%, 99%, bzw. 99,6% Umsatz nötig waren. Für diese Umätze wurde jeweils TR, ER und EV bestimmt.

Tabelle 6: Bestimmung von $TR_n$, $ER_n$ und $EV_n$:

| Umsatz $U_n$ | 95% | | 98% | | 99% | | 99,6% | |
|---|---|---|---|---|---|---|---|---|
| $TR_n$ [min]: | 93 | | 101 | | 106 | | 112 | |
| | ER[min] | $EV_{95}$ | ER[min] | $EV_{98}$ | ER[min] | $EV_{99}$ | ER [min] | $EV_{99,6}$ |
| Beispiel 1* | 350 | 3,75 | 640 | 6,33 | 802 | 7,94 | 1440 | 14,25 |
| Beispiel 2* | 280 | 3,00 | 410 | 4,06 | 530 | 5,24 | 840 | 8,31 |
| Beispiel 3 | 150 | 1,61 | 215 | 2,13 | 250 | 2,47 | 330 | 3,27 |
| Beispiel 4 | 275 | 2,95 | 350 | 3,46 | 400 | 3,96 | 500 | 4,95 |
| Beispiel 5 | 175 | 1,88 | 225 | 2,23 | 295 | 2,92 | 375 | 3,71 |

*Beispiel 1 und 2 sind nicht erfindungsgemäß.

Die Auswertung zeigt ganz deutlich, dass die Veresterung unter Begasungsbedingungen signifikant schneller abläuft, als die in konventionellen Rührkolben oder Festbettreaktoren unter Vakuum. Ferner wird ersichtlich, dass die Begasungsrate einen deutlichen Einfluss hat: In Beispiel 5 wurde die Begasungsrate im Vergleich zu Beispiel 4 von 5 $m^3/h$ auf 13 $m^3/h$ erhöht, wodurch EV im Durchschnitt um ein Drittel gesenkt wurde.

Beispiel 7: Wiederverwendung des Katalysators

Analog zu den Beispielen 3-5 werden mehrere Reaktionen zur Synthese von Myristylmyristat unter Wiederverwendung des Enzymkatalysators ohne Zwischenreinigung durchgeführt und der Umsatz nach 300 Minuten bestimmt (Ansatzgrößen jeweils 25,6 g Myristinsäure, 25,0 g Myristylalkohol und 0,20 g Novozym 435, Begasung 1500 mL/min Stickstoff. Es wurden 6 Wiederholungen durchgeführt.

Tabelle 7: Umsatzdaten zu Beispiel 7

| Wiederholung | Umsatz 300 min |
|---|---|
| 1 | 99,5 % |
| 2 | 99,4 % |
| 3 | 99,4 % |
| 4 | 99,3 % |
| 5 | 99,2 % |
| 6 | 99,4 % |

Es wird deutlich, dass auch bei wiederholter Verwendung des Katalysators keine signifikanten Umsatzeinbußen zu beobachten sind, die auf eine Desaktivierung des Katalysators schließen lassen könnte und dass vergleichbare Umsätze erreicht werden.

Beispiel 8: Herstellung von Decyl Cocoat

429 g Decanol (Aldrich, OHZ: 340 $mg_{KOH}$/g) und 553 g Cocosfettsäure (Edenor HK 8-18, Fa. Cognis, SZ: 271,4 $mg_{KOH}$/g) wurden in einem auf 60° beheizten Glasgefäß mit einem Innendurchmesser von 100 mm und einer Höhe von 500 mm auf 60 °C erhitzt. Durch eine am Gefäßboden angebrachte Glasfritte wurden 3 1/min Stickstoff in die Mischung eingetragen. Es wurden 3,9 g Novozym 435 zugegeben. Nach 9 Stunden hatte die Reaktionsmischung eine Säurezahl von 0,86 $mg_{KOH}$/g erreicht.

Beispiel 9: Herstellung eines Polyetheresters

418,7 g eines Polyethylen/Polypropylenglycols (Molgewicht 2790 g/mol, OHZ: 40,2 $mg_{KOH}$/g, Zusammensetzung: 52% Ethylenglycol, 48% Propylenglycol) und 55,3 g Undec-10-ensäure (2 Äquivalente, Aldrich, SZ: 304,4 4 $mg_{KOH}$/g) wurden in einem auf 60° beheizten Glasgefäß mit einem Innendurchmesser von 100 mm und einer Höhe von 500 mm auf 60 °C erhitzt. Durch eine am Gefäßboden angebrachte Glasfritte wurden 2 1/min Stickstoff in die Mischung eingetragen. Es wurden 9,5 g Novozym 435 zugegeben. Nach 23 Stunden hatte die Reaktionsmischung eine Säurezahl von 1,0 $mg_{KOH}$/g erreicht.

Beispiel 10: Herstellung eines Polyesters

404,6 g Adipinsäurediethylester (Aldrich) und 236,4 g 1,6-Hexandiol (Aldrich) wurden in einem auf 60° beheizten Glasgefäß mit einem Innendurchmesser von 100 mm und einer Höhe von 500 mm auf 60 °C erhitzt. Durch eine am Gefäßboden angebrachte Glasfritte wurden 2 1/min Stickstoff in die Mischung eingetragen. Es wurden 6,4 g Novozym 435 zugegeben. Nach 24 Stunden wurde per $^1$H-NMR ein Umsatz von 94% bBezogen auf Diethyladipat bestimmt. Eine GPC-Anylase (Agilent 1100, 1 mL/min THF, Polystyrol als Standard) zeigte die Bildung eines Polymers mit $M_w$ = 3819 g/mol und $M_N$ = 1854 g/mol.

**Patentansprüche**

1. Verfahren zur enzymatischen Synthese von Carbonsäureestern
mit Enzymimmobilisaten, hergestellt unter Einsatz von ganzen Zellen, ruhenden Zellen, gereinigten Enzymen oder Zellextrakten, die die entsprechenden Enzyme enthalten, oder Mischungen davon,
unter Verzicht auf Einsatz von Rührwerken im Reaktionsraum,
**dadurch gekennzeichnet, dass** Durchmischung der Reaktanden und Austrag des Reaktionswassers durch Einleiten eines Gases erfolgen unter Erreichung niedriger Effektivitätsverhältnisse EV, wobei eine Umsatzrate von 95% mit einem Effektivitätsverhältnis $EV_{95}$ < 3,0 erreicht wird, wobei sich das Effektivitätsverhältnis berechnet nach $EV_n = ER_n/TR_n$, mit $ER_n$ der effektiven Reaktionszeit, und $TR_n$ = theoretische Mindestreaktionszeit zum Erreichen des Umsatzgrads n, mit n = Umsatzgrad von 95%,.

2. Verfahren nach Anspruch 1, **dadurch gekennzeichnet, dass** der enzymatische Katalysator partikulär auf einem Trägermaterial kovalent oder nicht-kovalent fixiert ist.

3. Verfahren nach Anspruch 2, **dadurch gekennzeichnet, dass** das Trägermaterial eine mittlere Korngröße von größer als 0,5 μm aufweist.

4. Verfahren nach einem der Ansprüche 1 bis 3, **dadurch gekennzeichnet, dass** als Gas Luft, Magerluft, Sauerstoff, Stickstoff, Edelgase oder Kohlendioxid, bevorzugt Luft oder Stickstoff, eingesetzt werden.

5. Verfahren nach einem der Ansprüche 1 bis 4, **dadurch gekennzeichnet, dass** die Reaktion bei einer Temperatur von 20 °C bis 100 °C durchgeführt wird.

6. Verfahren nach einem der Ansprüche 1 bis 4, **dadurch gekennzeichnet, dass** 98% Umsatz mit einem Effektivitätsverhältnis $EV_{98}$ < 4,0 erreicht wird.

7. Verfahren nach einem der Ansprüche 1 bis 4, **dadurch gekennzeichnet, dass** 99% Umsatz mit einem Effektivitätsverhältnis $EV_{99}$ < 5,0 erreicht wird.

8. Verfahren nach einem der Ansprüche 1 bis 4, **dadurch gekennzeichnet, dass** 99,6% Umsatz mit einem Effektivitätsverhältnis $EV_{99,6}$ < 8,0 erreicht wird.

## Claims

1. Process for enzymatically synthesizing carboxylic esters
with enzyme immobilizates produced using whole cells, resting cells, purified enzymes or cell extracts which comprise the enzymes in question, or mixtures thereof
without using stirrers in the reaction chamber,
**characterized in that** mixing of the reactants and discharge of the water of reaction are effected by introducing a gas while achieving low effectiveness ratios EV, where a conversion rate of 95% is achieved with an effectiveness ratio $EV_{95}$ of < 3.0, where the effectiveness ratio is calculated according to $EV_n = ER_n/TR_n$ where $ER_n$ is the effective reaction time and $TR_n$ = theoretical minimum reaction time to attain the conversion level n, with n = conversion level of 95%.

2. Process according to Claim 1, **characterized in that** the enzymatic catalyst is fixed covalently or noncovalently in particulate form on a support material.

3. Process according to Claim 2, **characterized in that** the support material has a mean particle size of greater than 0.5 μm.

4. Process according to any one of Claims 1 to 3, **characterized in that** the gas used is air, lean air, oxygen, nitrogen, noble gases or carbon dioxide, preferably air or nitrogen.

5. Process according to any one of Claims 1 to 4, **characterized in that** the reaction is performed at a temperature of 20°C to 100°C.

6. Process according to any one of Claims 1 to 4, **characterized in that** 98% conversion is achieved with an effectiveness ratio $EV_{98}$ of < 4.0.

7. Process according to any one of Claims 1 to 4, **characterized in that** 99% conversion is achieved with an effectiveness ratio $EV_{99}$ of < 5.0.

8. Process according to any one of Claims 1 to 4, **characterized in that** 99.6% conversion is achieved with an effectiveness ratio $EV_{99.6}$ of < 8.0.

## Revendications

1. Procédé de synthèse enzymatique d'esters d'acides carboxyliques
avec des immobilisats d'enzymes, fabriqués en utilisant des cellules entières, des cellules au repos, des enzymes purifiées ou des extraits cellulaires contenant les enzymes appropriées ou des mélanges de ceux-ci,
sans utiliser d'agitateurs dans la chambre de réaction, **caractérisé en ce que** le mélange des réactifs et le déchargement de l'eau de réaction ont lieu par introduction d'un gaz pour atteindre des rapports d'efficacité EV faibles, un taux de conversion de 95 % étant atteint avec un rapport d'efficacité $EV_{95}$ < 3,0, le rapport d'efficacité étant calculé par $EV_n = ER_n/TR_n$, avec $ER_n$ le temps de réaction effectif, et $TR_n$ = le temps de réaction minimal théorique pour atteindre le degré de conversion n, avec n = degré de conversion de 95 %.

2. Procédé selon la revendication 1, **caractérisé en ce que** le catalyseur enzymatique est fixé sous forme particulaire sur un matériau support de manière covalente ou non covalente.

3. Procédé selon la revendication 2, **caractérisé en ce que** le matériau support présente une taille de particule moyenne supérieure à 0,5 μm.

4. Procédé selon l'une quelconque des revendications 1 à 3, **caractérisé en ce que** de l'air, de l'air pauvre, de l'oxygène, de l'azote, des gaz nobles ou du dioxyde de carbone, de préférence de l'air ou de l'azote, sont utilisés en tant que gaz.

5. Procédé selon l'une quelconque des revendications 1 à 4, **caractérisé en ce que** la réaction est réalisée à une température de 20 °C à 100 °C.

6. Procédé selon l'une quelconque des revendications 1 à 4, **caractérisé en ce qu'**une conversion de 98 % est atteinte avec un rapport d'efficacité $EV_{98}$ < 4,0.

7. Procédé selon l'une quelconque des revendications 1 à 4, **caractérisé en ce qu'**une conversion de 99 % est atteinte avec un rapport d'efficacité $EV_{99}$ < 5,0.

8. Procédé selon l'une quelconque des revendications 1 à 4, **caractérisé en ce qu'**une conversion de 99,6 % est atteinte avec un rapport d'efficacité $EV_{99,6}$ < 8,0.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102007031689
- EP 0413307 A


### In der Beschreibung aufgeführte Nicht-Patentliteratur


- Organikum. Wiley-VCH, 2004, 472
- **K. FABER.** Biotransformations in Organic Chemistry. Springer, 2000, 384
- *J. Am. Chem. Soc.,* 1999, vol. 121, 9487-9496
- *J. Mol. Catal. B,* 2005, vol. 35, 93-99
- *Eur. J. Lipid Sci. Technol.,* 2003, vol. 105, 601-607
- *J. Biotech.,* 2004, vol. 110, 209-217
- **VOET ; VOET.** Biochemie. Wiley-VCH, 1992, 329-331
- **L. CAO.** Carrier-bound Immobilized Enzymes: Principles, Application and Design. Wiley-VCH, 2005